# EUROPEAN PATENT APPLICATION

(11) **EP 2 198 895 A2**
(43) Date of publication of application: **23.06.2010**
(21) Application number: 09151740.9
(22) Date of filing: 30.01.2009
(51) Int. Cl.: A61L 27/18, A61L 27/36, A61L 27/56

(54) **Porous structures of microbial-derived cellulose for in vivo implantation**

(30) Priority: 19.12.2008 US 318038
(71) Applicant: Xylos Corporation, Langhorner, PA 19047 (US)
(72) Inventor: Serafica, gonzalo, Newtown, Pennsylvania 18940 (US); Weinberger, Lauren, Philadelphia, Pennsylvania 19130 (US)
(74) Representative: HOFFMANN EITLE

(57) **Abstract**

A transgenic plant transformed by a casein kinase Stress-Related Polypeptide (CKSRP) coding nucleic acid, wherein expression of the nucleic acid sequence in the plant results in increased tolerance to environmental stress as compared to a wild type variety of the plant. Also provided are agricultural products, including seeds, produced by the transgenic plants. Also provided are isolated CKSRPs, and isolated nucleic acid coding CKSRPs, and vectors and host cells containing the latter.

## Description

### FIELD OF THE INVENTION

This invention relates to polysaccharide materials and more particularly to microbial-derived cellulose having the porosity and containing pores of the desired size making it suitable for cellular infiltration during implantation and other desirable properties for medical and surgical applications. The invention also relates to the use of porous microbial -derived cellulose as tissue engineering matrices, human tissue substitutes, and reinforcing scaffolds for regenerating injured tissues and augmenting surgical procedures. The invention outlines various methods during and after fermentation to create porous microbial cellulose capable of allowing cell infiltration while preserving the physical properties of the microbial-cellulose.

### BACKGROUND OF THE INVENTION

Initially, the use of microbial-derived cellulose in the medical industry was limited to liquid loaded pads (U.S. Patent No. 4,588,400), wound dressings (U.S. Patent No. 5,846,213), and other topical applications (U.S. Patent No. 4,912,049). However, the use of microbial cellulose as an implantable medical device has also been explored and is well documented.

Oster et al. (U.S. Patent No. 6,599,518) described the use of microbial cellulose that has been solvent dehydrated prior to implantation. Mello et al. (Mello, L. R., et al., Duraplasty with Biosynthetic Cellulose: An Experimental Study, Journal of Neurosurgery, V. 86, 143-150 (1997)) published the use of microbial cellulose similar to the one described in (U.S. Patent No. 4,912,049) as a duraplasty material in an experimental animal study. Their results indicated that the dried form of the microbial-derived cellulose was adequate as a dural substitute. Damien et al. (U.S. Patent No. 7,374,775) also described the use of microbial cellulose sheets as an artificial dura mater implant.

Microbial cellulose wound dressings with large pores produced by introducing downward projecting cylindrical rods into a growing culture was discussed by Johnson & Johnson (US Patent No. 4,588,400 example 9). These pores were used to allow fluid movement through the dressings when used on exudating wounds. However, these pores were not over the entire surface of the dressing and were not the optimal size for cell and tissue ingrowth.

Recently, Falcao et al. (Falcao, S.C., et al., Biomechanical evaluation of microbial cellulose (Zoogloea sp.) and expanded polytetrafluorethylene membranes as implants in repair of produced abdominal wall defects in rats, Acta Cirurgica Brasileira, V. 23, 184-191 (2008)) evaluated the use of microbial cellulose sheets produced by the microorganism *Zoogloea sp.* in the repair of abdominal wall defects in a rat model. Infiltration of tissue from the abdominal wall was not observed at the interface between the microbial cellulose implant and abdominal wall in this study. Falcao et al. reported that the microporosity of the microbial cellulose was not sufficient to allow for the infiltration of host tissue into its structure. This suggests the need for microbial cellulose with a more macroporous structure to allow for infiltration of cellular elements and host tissue into the cellulose when implanted.

Svensson et al. (Svensson, A., et al., Bacterial cellulose as potential scaffold for tissue engineering of cartilage, Biomaterials, V. 26, 419-431 (2005)) reported on microbial cellulose as a potential for tissue engineering of cartilage and noted that it is desirable to have a scaffold material that has the porosity necessary to support cell ingrowth. Furthermore, Backdahl et al. (Backdahl, H., et al., Engineering microporosity in bacterial cellulose scaffolds, Journal of Tissue Engineering and Regenerative Medicine, V. 2, 320-330 (2008)) discussed a method to engineer microporosity into thin films (5 µm) of microbial cellulose by adding paraffin wax and starch particles of various sizes in a growing culture of *Acetobacter xylinum.* Only when using fused paraffin were pores observed throughout the thickness of the microbial cellulose samples.

Prior to the present invention, there has not be an acceptable process capable of creating implantable materials comprising microbial-derived cellulose of varying thicknesses with pore sizes that can allow for the cellular infiltration that is required for tissue incorporation.

An object of the present invention is to provide a porous microbial-derived implantable cellulose, wherein the material is capable of allowing cells to infiltrate the material while maintaining desired strength characteristics. Another object of the invention is to provide porous microbial-derived implantable cellulose, wherein the material is capable of *in vivo* implantation, and has desirable mechanical properties such as tensile strength, elongation and sutureability. Finally, the methods for producing these porous materials are also described and the potential uses for these porous materials are outlined.

### BRIEF DESCRIPTION OF THE FIGURES

Figure 1 shows the flow rate of filtered, deionized water through various porous and non-porous microbial cellulose samples.

Figure 2 shows the tensile strength of porous microbial-derived cellulose vs. non-porous material with various cellulose densities.

Figure 3 shows the suture pull-out strength of microbial-derived cellulose vs. non-porous material with various cellulose densities.

Figure 4 shows the tensile strength of samples perforated with a microneedle array on one or both sides either before or after drying in a controlled humidity chamber.

Figure 5 shows the suture pull-out strength of samples perforated with a microneedle array on one or both sides either before or after drying in a controlled humidity chamber.

Figure 6 shows tensile strength and suture pull-out strength of materials similar to those in J&J patent example 9.

Figure 7 shows a micrograph of a pore in a microbial cellulose sample.

Figure 8 shows a micrograph of a pore in a microbial cellulose sample.

Figure 9 shows a top down view of a pore after 3 weeks in a cell filtration study, before staining.

Figure 10 shows a top down view of a pore after 3 weeks in a cell filtration study, after staining.

Figure 11 shows a micrograph of a cross section of a pore with cells infiltrated the pore.

Figure 12 shows a micrograph of a cross section of a pore with cells infiltrating the pore.

### SUMMARY OF THE INVENTION

In one embodiment, the invention is directed to a microbial cellulose material comprising pores, wherein the pore diameter as determined by microscopy is in a range of about 50-500 microns.

In another embodiment, the invention is directed to a medical treatment comprising applying the afore-mentioned microbial cellulose material to a treatment site in a subject in need thereof.

In still another embodiment, the invention relates to a method for tissue repair, comprising administering to a subject in need thereof at a site in need of tissue repair the afore-mentioned microbial cellulose material and at least one cell seeded on the material prior to implanting.

The materials of the present invention comprise porous microbial-derived cellulose, particularly cellulose produced from static cultures of *Acetobacter xylinum* propagated in a nutrient media and incubated under controlled conditions. The porous cellulose film or pellicle can be produced during fermentation by having tapered pins or protrusions from below and/or above the air liquid interface of the liquid culture. The taper of such pins or rods is such that the area at the air liquid interface is greater than the area submerged in the liquid media. This allows the newly formed film or pellicle to be propelled below during cellulose synthesis and allow new films to grow on top of it. The pins or rods can be arranged in various configurations and distances between pins can be adjusted to the desired value. Suggested distances of less than 3 mm have been used. Depending on the desired thickness and cellulose density per unit area, the pellicles are allowed to incubate anywhere from 3 days to 1 month. After completion of the fermentation cycle, the pellicles are harvested and the pins and rods are removed. The pellicle is then chemically treated with sodium hydroxide to destroy pyrogens and viable microorganisms. The pellicle is bleached with hydrogen peroxide to whiten the cellulose. Following compression of each pellicle, the material may be further processed to make it degradable including chemically oxidizing the material with various oxidizing agents including nitrogen tetroxide and sodium periodate. The processed porous sheets are then dehydrated accordingly to produce the desired physical characteristics. The cellulose can be dehydrated by various means including processing the cellulose with a water-miscible organic solvent selected from the group consisting of methanol, ethanol, propanol, isopropanol, acetone and mixtures thereof. The use of supercritical drying with carbon dioxide may also be use to dehydrate the materials. Finally, the porous microbial material is cut, packaged, and gamma sterilized prior to use.

In another aspect of the invention, a second method for producing porous cellulose from microbial-derived cellulose can be accomplished post fermentation. The second method comprises the same steps of propagating cellulose-producing microbes in a nutrient media under controlled conditions but without using protruding pins in the bioreactor. Instead, the nonporous films produced after fermentation are harvested and processed similarly as above, including chemically treating the material to clean and remove any organisms present. A subsequent step involving oxidizing the said films may also be rendered in order to make the material bioresorbable using various oxidizing agents. The cleaned nonporous microbial cellulose films are then dehydrated to different levels prior to creating perforations or holes. The holes can be created using a microneedle array similar to a bed of nails or rollers with protruding pins at known distances from each other. The pins can vary in length from 200 microns to 10 mm depending on the thickness of the microbial cellulose sheet being perforated and whether the holes need to be straight through the sheet or just partially penetrating it to create a dual sided material with pores only on one side.

### DETAILED DESCRIPTION OF THE INVENTION

Unless otherwise specified, "a" or "an" means one or more.

Preferably, the microbial cellulose material is in a sheet form. Preferably, the thickness of the sheet is from about 0.1 mm to about 10 mm.

Preferably, the pore diameter is about 100-400 microns, even more preferably about 150-250 microns. Preferably, the pores partially penetrate the sheet on one side or completely extend through the sheet. Preferably, the pore density on the surface of the sheet is about 9 to about 81 pores per cm².

Preferably, the microbial cellulose material has a tensile strength of the sheet is from about 5 newtons to about 500 newtons.

Preferably, the microbial cellulose material has a suture resistance of the sheet is from about 0.5 newtons to about 50 newtons.

The porous microbial-derived cellulose of the present invention is synthesized by bacteria, preferably the bacteria *Acetobacter xylinum* (wild type), and is recovered from inoculation flasks and propagated via continued inoculation and incubation for linear growth in subsequent flasks and carboys of optimized media to attain the desired volume of microbial-derived cellulose. The media is comprised of nutrients such as sucrose, ammonium sulfate, sodium phosphate, magnesium sulfate, citric acid, acetic acid and trace elements resulting in a growth media having a pH of about 4.0 to 4.4. The sterilized media is inoculated from propagation cultures of *A. xylinum* and filled into bioreactor trays at the appropriate volume to produce various sheets of microbial cellulose with different cellulose fiber densities that in turn produce sheeted material with varying tensile and suture strengths.

The bioreactor trays are then fitted with protruding pins from both above and below the liquid interface prior to sealing and incubating in a controlled environment at 30° C ± 2° until growth of the pellicle of microbial-derived cellulose is complete. The pellicles are removed from the bioreactor trays and are chemically treated to remove bacterial by-products and residual media. A caustic solution, preferably sodium hydroxide at a preferable concentration of about 0.1M to 4M, is used to remove viable organisms and pyrogens (endotoxins) produced by bacteria from the pellicle. The treated pellicles are then rinsed with filtered water to reduce microbial contamination (bioburden).

The cleaned microbial cellulose sheet can be further processed to make it bioresorbable by using various means including chemically oxidizing the material with oxidizing agents such as nitrogen tetroxide and sodium periodate. The level of oxidation can be adjusted depending on the desired rate of resorption of the materials during implantation and can range from 5% to 100% oxidized. After the oxidation process, the oxidized films are rinsed to remove excess oxidizing agents and are soaked in various solvents such as methanol to prepare it for subsequent dehydration process.

In a controlled environment, the pellicles may be compressed to a desired thickness. It is the thickness of the compressed film that achieves the final desired density of the microbial-derived cellulose. The original fill volumes as well as the compression steps are integral to the present invention to attain the desired density that affects the strength, integrity, and function of the cellulose. As mentioned before, the present invention may be further dehydrated using various drying methods including using solvents and super critical fluids. Depending on the desired level of dehydration, the solvent treated films are exposed to one or more applications of the organic solvent and may be subsequently compressed to the desired thickness in a controlled environment. The solvent is removed under controlled conditions. The microbial cellulose materials can also be immersed in supercritical fluids such as carbon dioxide and then dehydrated by releasing the pressure. The super critical drying creates materials with preserved open structures and minimizes crystallization of the cellulose fibers by reducing the surface tension between fibers during drying and solvent removal.

In a controlled environment, the films can be cut to various shapes and sizes. An additional step using a microneedle array may be performed to introduce pores with diameters of 100-500 microns. The density of the holes per unit area can be controlled as well as the distance of each pore from one another depending on the desired final physical characteristics and intended application. The holes are preferably arranged in a regular pattern, for example in a chess board pattern with preferred distances between the nearest neighbors in the range of 0.5 to 5 mm.

It is possible for each unit to be packaged in a waterproof double-pouch system and sterilized by exposure to gamma irradiation at a dose level up to 35 kGy, but preferably a lower dose would be used. The gamma dose is determined by the bioburden level of the non-sterile material as described in ISO 11137 Sterilization of Health Care Products-Requirements for validation and routine control-Radiation Sterilization.

The ability of the present inventive microbial-derived cellulose to be used in surgical procedures requires that the material is safe and effective for its intended purpose and achieves sufficient biocompatibility. The ability of the present invention to withstand depyrogenation and sterilization processes is necessary toward producing an implantable medical device for general and plastic surgery. Often, biomedical polymers have lower thermal and chemical stability than other materials such as metals, ceramics and synthetics; therefore, they are more difficult to sterilize using conventional methods. For any material used as an implantable medical device, it must be free from endotoxins (non-pyrogenic), microorganisms and other possible contaminants that will interfere with the healing process and cause harm to the recipient.

The present invention undergoes depyrogenation by using a heated caustic solution (0.1M to 4M sodium hydroxide) known to destroy endotoxins that may be present due to bacteria or cross-contamination from materials exposed to pyrogens. The material is then gamma irradiated at doses sufficient to destroy microorganism contamination by pre-determined sterility assurance levels based on bioburden levels (the amount of microorganisms typically present on the non-sterile material). Samples are gamma irradiated at a dose of about 35 kGy. It can be concluded that the material can be depyrogenated with a strong alkaline sodium hydroxide solution at an elevated temperature and that it can withstand gamma sterilization without any significant affect to mechanical properties.

The inventive microbial-derived cellulose can be used in tissue augmentation which involves implantation of the subject microbial-derived cellulose material for general as well as plastic surgery applications. Examples of general and plastic surgical uses include but are not limited to, general soft tissue augmentation, pelvic floor reconstruction, bladder neck suspension, hernia repair, inguinal hernia patch and rotator cuff reinforcement

Another use of the present inventive porous cellulose material involves their application as a tissue engineering matrix for cell seeding. Suture retention is critical for implantable medical articles to secure and maintain position during surgery, healing and function. The surgeon must rely on the ability of the implantable material to not only accept suture without tearing during needle insertion, but to also retain the suture without tearing away from the sutured edge of the implant.

Medical devices intended for implant must meet various criteria to comply with either the U.S. Food and Drug Administration (FDA) regulations or the International Organization for Standardization (ISO) requirements in order to be deemed fit for their intended use. Cytotoxicity studies are considered relevant to prove that the implant device is safe/biocompatible with human tissue. *In vitro* biocompatibility studies, based on the International Organization for Standardization 10993: Biological Evaluation of Medical Devices, Part 5: Tests for Cytotoxicity: *in vitro* Methods Guidelines, were conducted on the present invention to determine the potential for Cytotoxicity.

The mechanical properties of the microbial-derived cellulose relates to tensile strength, % elongation and suture retention. The material is considered multidirectional, therefore no regard was made for the direction of the cutting.

The following examples are given to illustrate the present invention. It should be understood, however, that the invention is not to be limited to the specific conditions or details described in these examples. Throughout the specification, any and all references are specifically incorporated into this patent application by reference.

### EXAMPLE 1 - Manufacture of Implantable Microbial-Derived cellulose

This example is directed to a preparation of porous microbial-derived cellulose films produced by *A*, *xylinum* within a controlled environment to minimize bioburden (microorganism contamination). From a propagation vessel, sterilized media was inoculated with *A. xylinum*, filled into bioreactor trays that can be fitted with protrusions from above and below the air liquid interface The size of the protruding pins vary depending on the desired pores and can range from 200 microns to 10 mm. The fill volume ranging 180-550 g, and incubated for 10-35 days depending on the desired pellicle thickness and eventual cellulose density. The pellicles were extracted from the trays and then underwent chemical processing (depyrogenation) in a tank of 8% sodium hydroxide which was heated to about 90°C to 95°C for about one hour. The pellicles then underwent a continuous rinse with filtered water until the pH was below 10.0. The material was treated with 0.25% hydrogen peroxide at 44°C to 45°C for about 30 minutes when the films were observed to be adequately bleached. The films were then rinsed with filtered water until the hydrogen peroxide level was below 1000 ppm. The films were compressed within a pneumatic press to yield a pellicle having a thickness of approximately 2 mm, water content on the order of 95%, and microbial-derived cellulose content approaching 5%.

The pressed films were subsequently soaked in several solvent baths to reduce the water content and increase the solvent concentration to greater than 95%. The films were again compressed within a pneumatic press prior to dehydration to increase the cellulose content of the films to greater than 90% in a controlled humidity chamber. The films were removed from the oven and cut into various sizes and shapes. The excess material was assayed for residual moisture and the material was partially rehydrated if necessary prior to creating additional pores using a microneedle array. Each unit was placed in an "inner" pouch, sealed, then placed within an "outer" pouch and sealed. The pouches were then sterilized via gamma irradiation at a dose in the range of 35 kGy. The sterilized samples made in accordance with the present invention were used for various tests, inclusive of tensile strength, elongation, and suture retention (pull-out).

### EXAMPLE 2 - Permeability testing

Samples with fill volumes of 30 g, 110 g, 180 g, and 530 g were prepared according to Example 1. Samples were either left non-porous, or 100-300 µm diameter pores were created using a microneedle array on one side (single sided) or both sides (double sided) of the sample. Furthermore, samples were either left hydrated following the final compression or were further dried in a controlled drying (CD) chamber.

Additional samples were prepared similarly to those in example 9 of a Johnson & Johnson patent (US. Patent No. 4,788,146), where 1/8 inch diameter rods were introduced into a 250 g growing culture, resulting in square pattern of 7x7, 1/8 inch diameter pores.

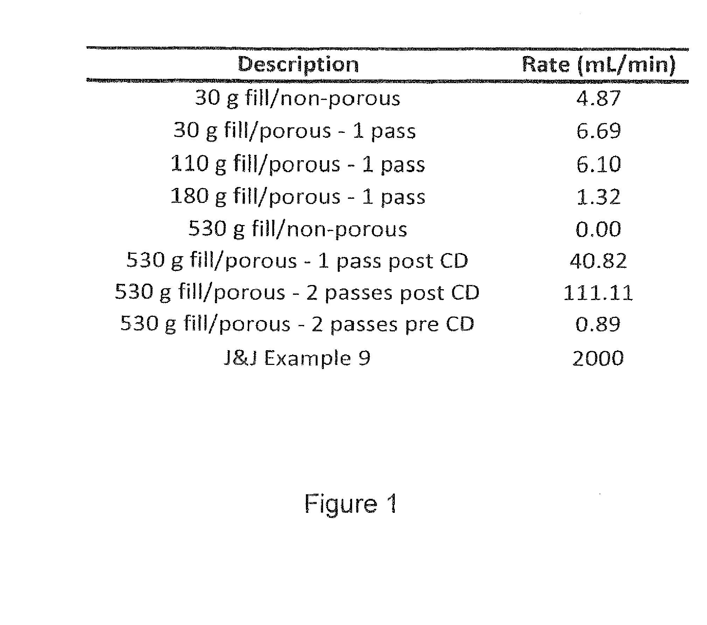

The specimens were placed in a vacuum filter holder and 100 mL of filtered, deionized water was filtered through the sample using a vacuum pump with a maximum vacuum of 21.3 Hg. The time for the water to filter through each sample was recorded and the filtration rate in mL/min was calculated. If the sample did not allow water to pass through, filtration was stopped at approximately 20 minutes. The filtration rates of filtered, deionized water through the various materials are shown in Figure 1.

### EXAMPLE 3 - Comparison of Mechanical Properties of the Porous and Non porous microbial cellulose

The mechanical properties of three sample sets with 100-300 µm diameter pores were tested. The first sample set consisted of samples with fill volumes of 30 g, 110 g, and 180 g were prepared according to Example 1. After the final dehydration press, samples remained hydrated and pores were created using a microneedle array. The second set consisted of samples with a fill volume of 530 g prepared according to Example 1. Pores on one side (single sided) or both sides (double sided) were created using a microneedle array either before control drying or after the final rehydration step following drying in a controlled humidity chamber. The third samples set contained porous and non-porous samples prepared as in Example 2 following the Johnson & Johnson patent example 9.

### Tensile Strength Test

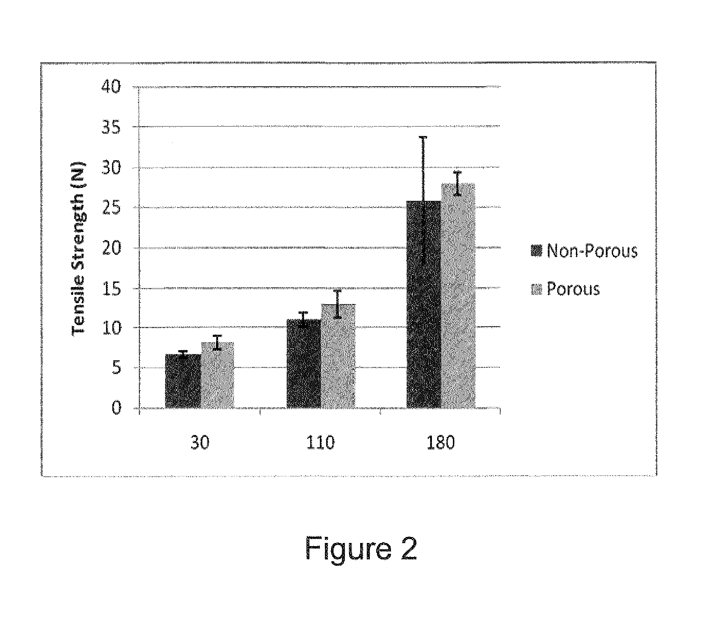

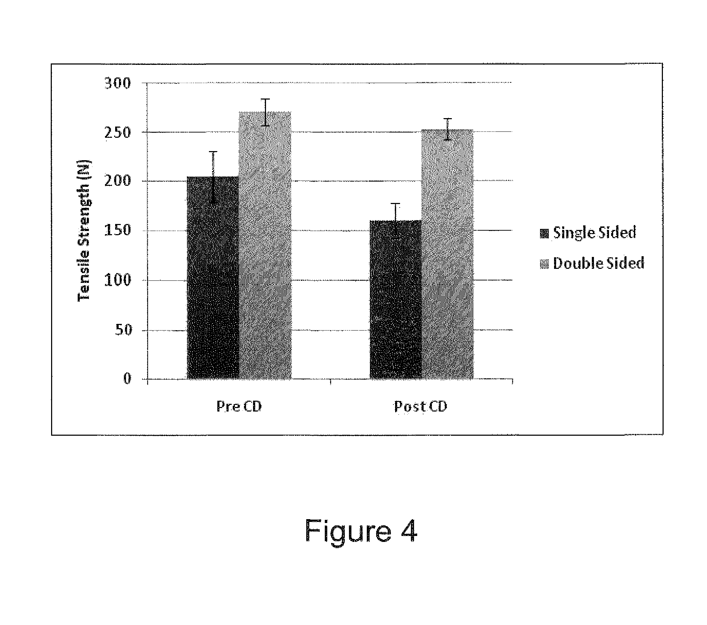

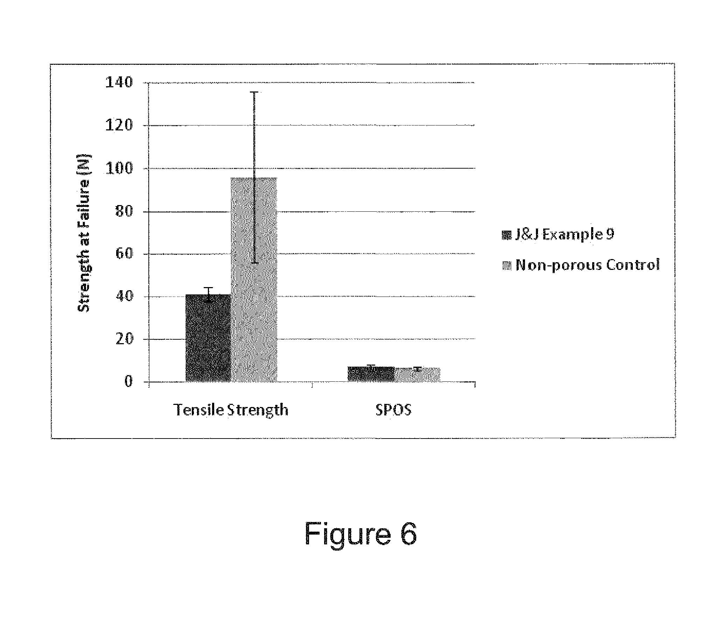

Test specimens were cut from larger pieces (4cm x 5cm) into 1cm x 4cm test strips and mounted into pneumatic clamps on a United Tensile Tester (Model SSTM-2kN) with a calibrated 100 N load cell. The gauge length of the specimens was 25 mm. The specimens were subjected to displacement at a rate of 300 mm/minute until the specimen failed completely. Failure was determined from the force-displacement curve as the maximum force (N). Tensile testing results are shown in Figures 2, 4, and 6.

### Suture Pull-Out Strength (SPOS) Test

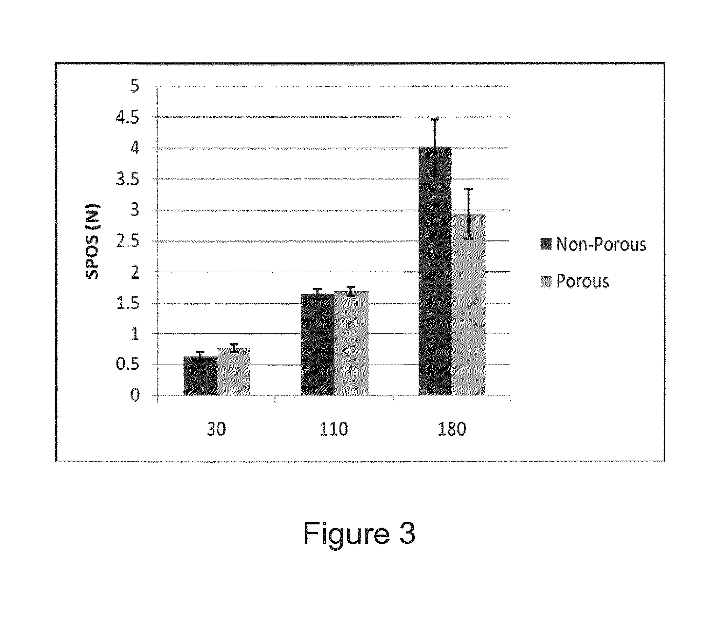

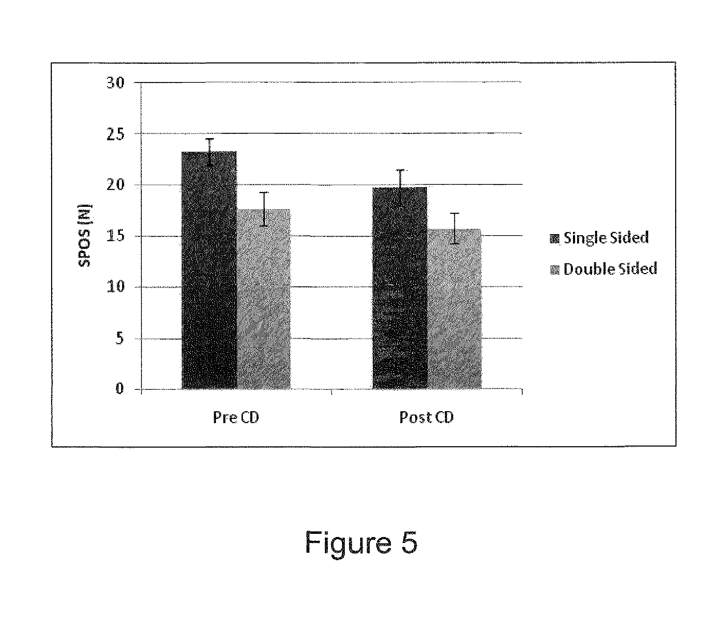

Specimens were cut from larger pieces (4cm x 5cm) into 1cm x 4cm test strips. The suture pull-out test specimens were prepared with a 2-0 polypropylene suture (Prolene, Ethicon Inc.) using a curved needle, immediately prior to testing. A single stitch was placed approximately 5 mm from each side and 4 mm from the bottom edge of the specimen. The suture thread was cut to approximately 10 cm. The top end of the specimen was mounted into pneumatic clamps on a United Tensile Tester (Model SSTM-2kN) with a calibrated 100N load cell and a gauge length of 60 mm. Both ends of the suture were mounted into the lower set of pneumatic clamps. The specimens were subjected to a displacement of 300 mm/minute until the specimen failed completely. SPOS results are shown in Figures 3, 5, and 6.

### EXAMPLE 4 - Microscopic examination

Samples with fill volumes of 530 g were made according to Example 1. 100-300 µm diameter pores were made using a microneedle array after the final drying step and the samples were not rehydrated.

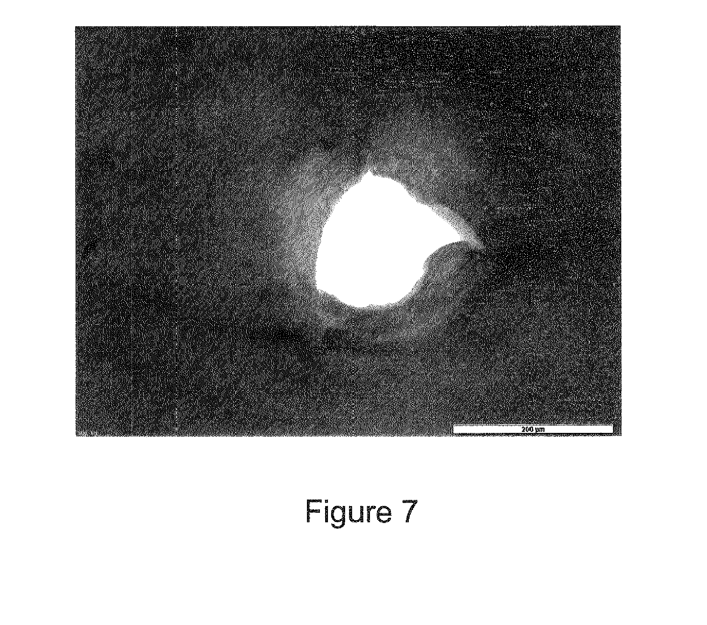

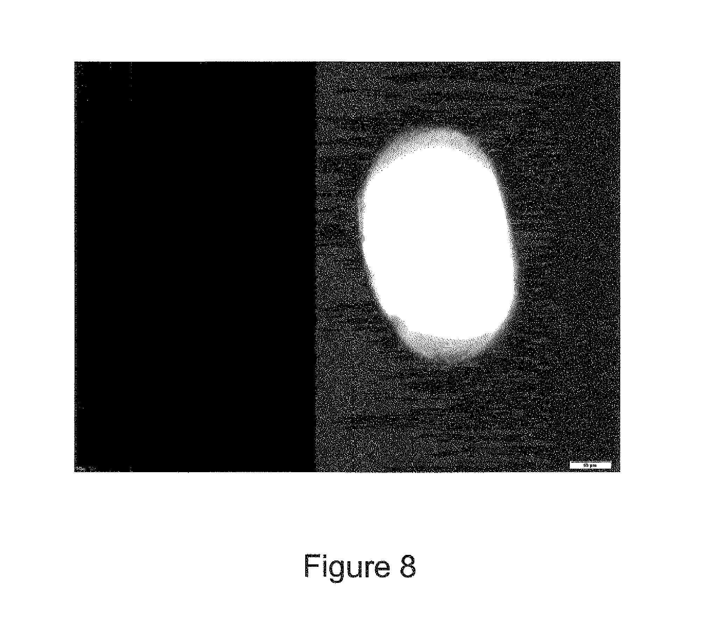

Microscopic evaluation of the samples was performed using an Olympus light microscope (Model BX41TF). At 10X magnification, the various pores are visible and depending on the specific processing, the pores range from 100-250 micron in diameter. The distance between each of the distinct pores was roughly 3 mm. Micrographs of the pores are shown in Figures 7 and 8.

### EXAMPLE 5 - Cell Infiltration

Samples with fill volumes of 530 g were made according to example 1. After the final rehydration step, a microneedle array was used to produce pores with diameters of approximately 100 to 300 µm.

The porous microbial cellulose films were pre-soaked in cell culture media for approximately 15-30 min. The test article was then placed in a six well plate and additional cell culture media was added as necessary. Each of the wells were then inoculated with a known number of cells and subsequently incubated at 37degrees centigrade in a 5% CO₂ chamber. The media was changed in each of the wells every 2-3 days and samples of the porous cellulose sheet was taken at different time points for up to 3 weeks.

### Microscopic Evaluation

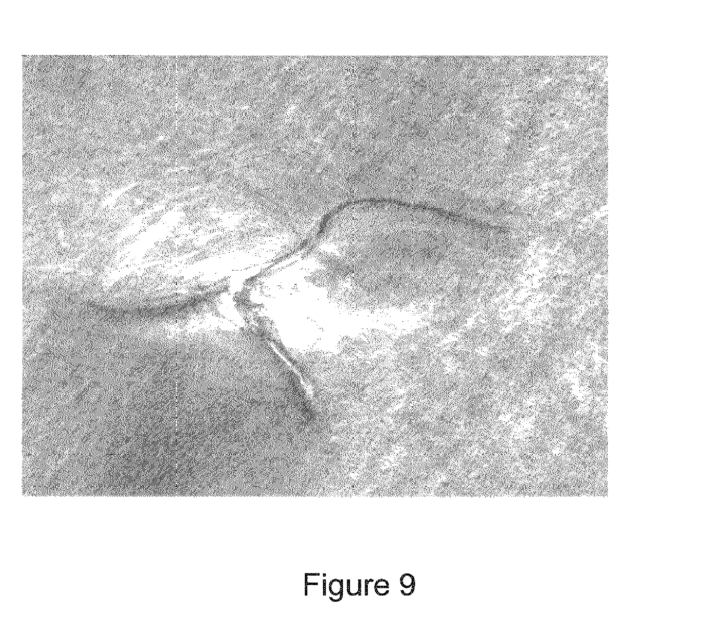

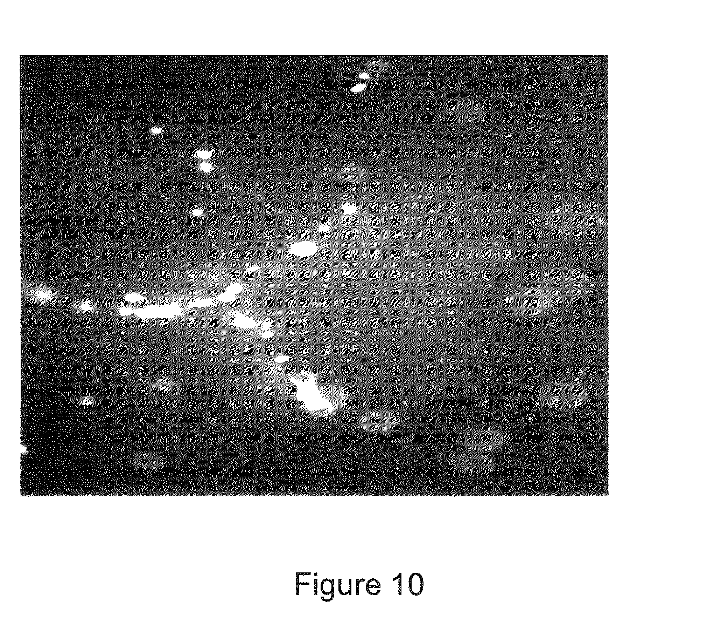

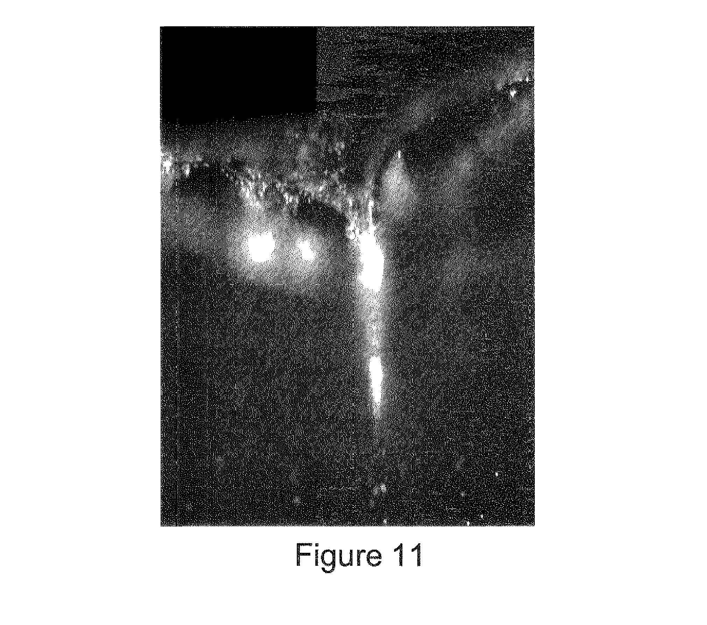

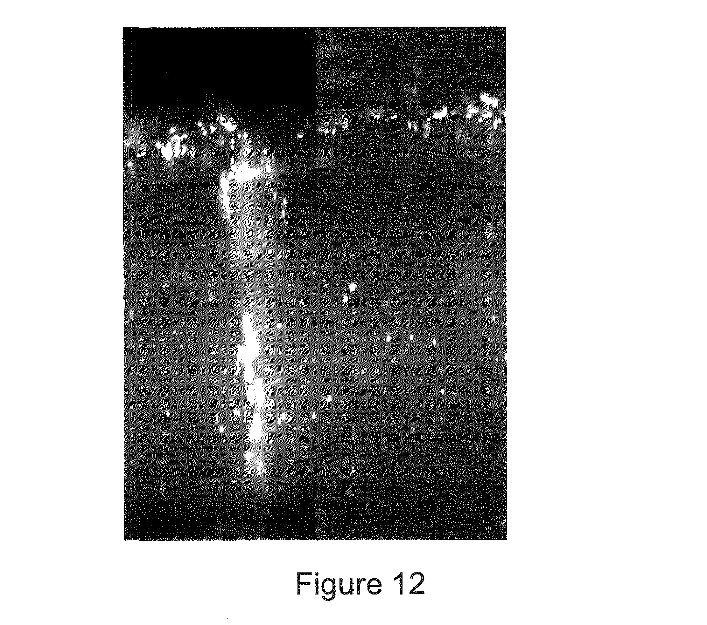

The cellulose article taken at different time points was then prepared for microscopic evaluation. The cut sections were placed on a glass slide and stained to identify the cells. The stained cells were shown to migrate from the top of the material into the pores. The cell infiltration experiment demonstrates the migration of chondrocytes (about 10 microns in length) into pore channels measuring approximately 150 microns in diameter, showing that the pores allow for cell infiltration into the material. Micrographs of the cell infiltration samples are shown in Figures 9, 10, 11, and 12.

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention.

All of the publications, patent applications and patents cited in this specification are incorporated herein by reference in their entirety.

## Claims

1. A microbial cellulose material comprising pores, wherein the pore diameter as determined by microscopy is in a range of about 50-500 microns.

2. The material of claim 1, wherein the microbial cellulose material is in a sheet form.

3. The material of claim 1 or 2, wherein the pore diameter is about 100-400 microns.

4. The material of claim 3, wherein the pore diameter is about 150-250 microns.

5. The material of one or more of the preceding claims, wherein the pores partially penetrate the sheet on one side or completely extend through the sheet.

6. The material of one or more of the preceding claims, wherein a thickness of the sheet is from about 0.1 mm to about 10 mm.

7. The material of one or more of the preceding claims, wherein pore density on the surface of the sheet is about 9 to about 81 pores per cm².

8. The material of one or more of the preceding claims, wherein a tensile strength of the sheet is from about 5 newtons to about 500 newtons.

9. The material of one or more of the preceding claims, wherein a suture resistance of the sheet is from about 0.5 newtons to about 50 newtons.

10. The microbial cellulose material of one or more of claims 1 to 9 for use in a medical treatment.

11. The material of claim 10, wherein the medical treatment is tissue augmentation, implantation of a tissue engineering matrix, hernia repair, pelvic floor reconstruction, bladder neck suspension, inguinal hernia patch, rotator cuff reinforcement or implantable cell and other bioactive agent delivery.

12. The material of claim 10, wherein the medical treatment is one of tissue repair.

13. The material of claim 12, wherein the microbial cellulose material and at least one cell seeded on the material are administered to a subject in need thereof at a site in need of tissue repair prior to implanting.
